(19) Europäisches Patentamt
European Patent Office
Office européen des brevets

(11) Veröffentlichungsnummer : **0 337 116 B1**

(12) # EUROPÄISCHE PATENTSCHRIFT

(45) Veröffentlichungstag der Patentschrift : **06.05.92 Patentblatt 92/19**

(51) Int. Cl.$^5$ : **C08G 18/79,** C08G 18/02, C07D 229/00, C07D 251/34

(21) Anmeldenummer : **89104169.1**

(22) Anmeldetag : **09.03.89**

(54) **Verfahren zur Herstellung von Uretdion- und Isocyanuratgruppen aufweisenden Polyisocyanatgemischen.**

Die Akte enthält technische Angaben, die nach dem Eingang der Anmeldung eingereicht wurden und die nicht in dieserPatentschrift enthalten sind.

(30) Priorität : **19.03.88 DE 3809261**

(43) Veröffentlichungstag der Anmeldung : **18.10.89 Patentblatt 89/42**

(45) Bekanntmachung des Hinweises auf die Patenterteilung : **06.05.92 Patentblatt 92/19**

(84) Benannte Vertragsstaaten : **BE DE FR GB IT NL**

(56) Entgegenhaltungen :
**EP-A- 0 010 589**
**EP-A- 0 173 252**
**DE-A- 1 670 720**

(73) Patentinhaber : **BAYER AG**
**W-5090 Leverkusen 1 Bayerwerk (DE)**

(72) Erfinder : **Pedain, Josef, Dr.**
**Haferkamp 6**
**W-5000 Köln 80 (DE)**
Erfinder : **König, Klaus, Dr.**
**Zum Hahnenberg 40**
**W-5068 Odenthal (DE)**
Erfinder : **Dell, Winfried, Dr.**
**Bruchhauser Strasse 99**
**W-5090 Leverkusen 3 (DE)**

**EP 0 337 116 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein neues Verfahren zur Herstellung von Uretdion- und Isocyanuratgruppen aufweisenden Polyisocyanaten, d.h. Polyisocyanatgemischen, durch Oligomerisierung eines Teils der Isocyanatgruppen von Hexamethylendiisocyanat (nachstehend "HDI" genannt) unter Verwendung von Katalysatoren, die sowohl die Dimerisierung (Uretdionbildung) als auch die Trimerisierung (Isocyanuratbildung) beschleunigen.

Die Herstellung von Uretdion- und Isocyanuratgruppen aufweisenden Polyisocyanatgemischen durch Di- und/oder Trimerisierung eines Teils der Isocyanatgruppen von organischen Polyisocyanaten unter Verwendung von Phosphor enthaltenden Katalysatoren ist bereits bekannt. Diese Gemische sind bekanntlich wertvolle Ausgangsmaterialien für die Herstellung von Polyurethankunststoffen. Die bekannten Herstellungsverfahren für die entsprechenden HDI-Derivate (vgl. z.B. DE-OS 1 670 720 und DE-OS 3 432 081) sind jedoch nicht optimal für eine großtechnische Herstellung geeignet. Die Nachteile dieser vorbeschriebenen Verfahren sind vor allem in der langen Dauer der Umsetzung und den verhältnismäßig hohen Mengen an Katalysatoren zu sehen, die zur Folge haben, daß eine entsprechend hohe Menge an Desaktivatoren eingesetzt werden muß, so daß das Endprodukt einen verhältnismäßig hohen Anteil an unerwünschten, die Eigenschaften eines Polyurethankunststoffes nachteilig beeinflussenden Fremdstoffen enthält.

Beim Verfahren der DE-OS 34 37 635 werden neben den bekannten Katalysatoren beträchtliche Mengen an Alkoholen als Cokatalysatoren verwendet, was selbstverständlich mit dem Nachteil verbunden ist, daß durch die zwischen Isocyanatgruppen und Hydroxylgruppen ablaufende Additionsreaktion wertvolle Isocyanatgruppen verbraucht, d.h. vernichtet werden.

Es war daher die der Erfindung zugrunde liegende Aufgabe, ein neues Verfahren zur Herstellung von Uretdion- und Isocyanuratgruppen aufweisenden Polyisocyanatgemischen auf Basis von HDI zur Verfügung zu stellen, welches nicht mit den genannten Nachteilen behaftet ist.

Diese Aufgabe konnte erfindungsgemäß dadurch gelöst werden, daß als Ausgangsmaterial praktisch Kohlendioxid freies HDI verwendet wird. Hierdurch ist es möglich, die Reaktionsdauer auf weniger als einen Arbeitstag, also weniger als 10 Stunden, zu begrenzen und gleichzeitig das Verfahren unter Verwendung von minimalen Mengen an Katalysatoren und ohne Mitverwendung von hohen Mengen an Isocyanatgruppen verbrauchenden Cokatalysatoren durchzuführen.

Gegenstand der Erfindung ist ein Verfahren zur Herstellung von Isocyanuratgruppen und Uretdiongruppen aufweisenden Polyisocyanatgemischen durch Oligomerisierung eines Teils der Isocyanatgruppen von Hexamethylendiisocyanat unter Verwendung von Trialkylphosphinen und/oder peralkylierten Phosphorigsäuretriamiden als die Dimerisierung und die Trimerisierung von Isocyanatgruppen beschleunigende Katalysatoren bis zum gewünschten Oligomerisierungsgrad, Beendigung der Reaktion durch Zugabe eines Katalysatorgifts und Entfernung von nicht umgesetztem Hexamethylendiisocyanat bis auf einen Restgehalt von max. 0,5 Gew.-%, dadurch gekennzeichnet, daß man das zum Einsatz gelangende Hexamethylendiisocyanat vor der Zugabe des Katalysators bis auf einen Restgehalt von weniger als 10 ppm (Gewicht) von Kohlendioxid befreit.

Von erfindungswesentlicher Bedeutung ist die Verwendung von praktisch Kohlendioxid-freiem HDI als Ausgangsmaterial. Das erfindungsgemäß zum Einsatz gelangende HDI weist einen Gehalt an Kohlendioxid von weniger als 10 ppm (Gewicht) und bevorzugt von weniger als 5 ppm (Gewicht) auf.

Technisches, durch Destillation gereinigtes HDI, wie es bislang auch zur Herstellung von Isocyanuratgruppen aufweisenden Polyisocyanaten eingesetzt wurde, weist beträchtliche Mengen (ca. 20 ppm bis 100 ppm (Gewicht)) an Kohlendioxid auf. Kohlendioxid kann bei der Herstellung in das HDI gelangen, beispielsweise bei der Phosgenierung von Kohlensäuresalzen des Hexamethylendiamins. Es kann beim Lagern aus der Luft aufgenommen werden, und es kann durch chemische Reaktion der NCO-Gruppen, z.B. durch Carbodiimidbildung oder mit einer Spur Feuchtigkeit, entstehen. Frisch durch Vakuumdestillation gereinigtes HDI enthält nach 24 Stunden im verschlossenen Gefäß beispielsweise 40 ppm Kohlendioxid. Über einen Zeitraum von ca. 6 Monaten gelagertes HDI kann, falls das Gebinde während der Lagerzeit geöffnet wurde, bis zu 0,6 Gew.-% Kohlendioxid enthalten.

Kohlendioxid reagiert bekanntlich mit Uretdiongruppen zu unerwünschten Nebenprodukten, die Oxadiazinon-Gruppen enthalten. Auf die Bildung von Nebenprodukten durch Kohlendioxid wird bereits in DE-OS 16 70 720 hingewiesen. Auch in der EP-A-O 010 589 wird darauf hingewiesen, daß bei der Herstellung von Isocyanuratgruppen aufweisenden Polyisocyanaten möglichst reines Ausgangsdiisocyanat (HDI) einzusetzten ist.

Den zitierten Vorveröffentlichungen kann keinerlei Hinweis entnommen werden, daß eine durch eine Vakuumdestillation überhaupt nicht mehr realisierbare weitere Reduzierung des Kohlendioxidgehalts auf unter 10 ppm noch mit irgendeinem meßbaren technischen Effekt verbunden sein könnte.

Die Entfernung von Kohlendioxid aus HDI kann durch Ausblasen mit Reinstickstoff oder Edelgas, z.B. mit Argon, erfolgen, z.B. bei 0 bis 120°C, vorzugsweise 0 bis 70°C und besonders bevorzugt 30 bis 50°C. Auch

eine höhere Temperatur kann angewendet werden, bietet aber keine entscheidenden Vorteile. Auch mit einer Destillation am Stickstoff- oder Edelgasstrom kann Kohlendioxid entfernt werden. Die Art der Entfernung von Kohlendioxid ist nicht entscheidend für die Durchführung des erfindungsgemäßen Verfahrens. Allerdings ist, wie gesagt, durch eine einfache Destillation gegebenenfalls unter vermindertem Druck eine praktisch restlose Entfernung von Kohlendioxid unter einen Gehalt von 20 ppm im allgemeinen nicht möglich.

Als Katalysatoren werden beim erfindungsgemäßen Verfahren tert.-Phosphine oder peralkylierte Phosphorigsäuretriamide eingesetzt. Selbstverständlich können auch Gemische aus tert.-Phosphinen und peralkylierten Phosphorigsäuretriamiden verwendet werden, obwohl dies weniger bevorzugt ist. Geeignete tert.-Phosphine sind insbesondere aliphatischer araliphatische oder gemischt aliphatisch-aromatische tert.-Phosphine des Molekulargewichtsbereichs 76 bis 500, d.h. dieser Definition entsprechende Verbindungen der allgemeinen Formel

$$\begin{array}{c} R'-P-R'' \\ | \\ R''' \end{array} ,$$

in welcher

R′, R″ und R‴ für gleiche oder verschiedene Reste stehen und Alkylgruppen mit 1 bis 10, vorzugsweise 2 bis 8 Kohlenstoffatomen, Aralkylgruppen mit 7 bis 10, vorzugsweise 7 Kohlenstoffatomen oder Arylgruppen mit 6 bis 10, vorzugsweise 6 Kohlenstoffatomen bedeuten, mit der Maßgabe, daß höchstens einer der Reste für eine Arylgruppe und vorzugsweise mindestens einer der Reste für eine Alkylgruppe steht, oder für welche zwei der Reste aliphatischer Natur sind und, miteinander verknüpft, zusammen mit dem Phosphoratom einem Phosphor als Heteroatom enthaltenden Ring mit 4 bis 6 Ringgliedern bilden, in welchem Falle der dritte der gennanten Reste für eine Alkylgruppe mit 1 bis 4 Kohlenstoffatomen steht.

Beispiele geeigneter tert.-Phosphine sind Triethylphosphin, Dibutylethylphosphin, Tri-n-propylphosphin, Tri-iso-propylphosphin, Tri-tert.-butylphosphin, Tribenzylphosphin, Benzyldimethylphosphin, Dimethylphenylphosphin, Tri-n-butylphosphin, Tri-iso-butylphosphin, Tri-amylphosphin, Trioctylphosphin oder Butyl-phosphacyclopentan. Tri-(n-butyl)-phosphin ist besonders gut als Katalysator für das erfindungsgemäße Verfahren geeignet.

Als Katalysatoren geeignete peralkylierte Phosphorigsäuretrimamide sind beliebige organische Verbindungen der allgemeinen Formel

$$P(NR_2)_3,$$

in welcher

die einzelnen Reste R für gleiche oder verschiedene, vorzugsweise gleiche Alkylreste mit 1 bis 10, vorzugsweise 1 bis 4 Kohlenstoffatomen, Aralkylreste mit 7 bis 10, vorzugsweise 7 Kohlenstoffatomen oder Cycloalkylreste mit 4 bis 10, vorzugsweise 6 Kohlenstoffatomen stehen. Aus dieser Definition der Reste R wird ersichtlich, daß der Begriff "peralkylierte" im Rahmen der Erfindung breit dahingehend zu interpretieren ist, daß nicht nur echte Alkylreste sondern auch Cycloalkyl- und Aralkylreste als mögliche Substituenten des Stickstoffatoms in Betracht kommen. Vorzugsweise handelt es sich jedoch bei den erfindungsgemäß als Katalysatoren einzusetzenden peralkylierten Phosphorigsäuretriamiden um solche der genannten allgemeinen Formel, für welche alle Reste R für Alkylreste mit 1 bis 4 Kohlenstoffatomen, vorzugsweise für Methylreste stehen. Das permethylierte Phosphorigsäuretriamid, d.h. Tris-(dimethylamino)-phosphin ist der bevorzugte beim erfindungsgemäßen Verfahren zum Einsatz gelangende Katalysator aus der Klasse der Phosphorigsäuretriamide.

Bezogen auf die Gesamtmenge HDI werden die genannten Katalysatoren in einer Menge von 0,01 bis 2, bevorzugt von 0,1 bis 1 und insbesondere von 0,1 bis 0,5 Gew.-% eingesetzt. Das erfindungsgemäße Verfahren wird vorzugsweise lösungsmittelfrei durchgeführt, was die Mitverwendung von üblichen Lacklösungsmitteln bei der Oligomerisierungsreaktion, beispielsweise von Estern wie Butylacetat oder Ethoxyethylacetat, Ketonen wie Methyl-isobutyl-keton oder Methylethylketon oder Kohlenwasserstoffen wie Xylol bzw. von Gemischen derartiger Lösungsmitteln jedoch nicht ausschließt. Da im Anschluß an die Reaktion jedoch nicht umgesetztes HDI entfernt wird, würde die Mitverwendung derartiger Lösungsmittel während der Reaktion lediglich auf einen überflüssigen, zusätzlichen Aufwand hinauslaufen.

Die Durchführung des erfindungsgemäßen Verfahrens kann beispielsweise entsprechend den in DE-OS 1 670 720, DE-OS 3 432 081 oder DE-OS 3 437 635 beschriebenen Verfahren erfolgen. So kann beispielsweise das weitgehend von Kohlendioxid befreite HDI in einem geeigneten Rührgefäß vorgelegt werden, worauf sich die Zugabe des Katalysators bei 0 bis 100°C, vorzugsweise 20 bis 70°C, anschließt, wonach die Reaktionsmasse durch Kühlen bzw. Erwärmen, vorzugsweise unter Rühren, innerhalb des Temperaturbereichs von 0 bis 100°C, vorzugsweise 20 bis 70°C, gehalten wird. Es ist vorteilhaft, während der gesamten Dauer der Umsetzung einen Strom von trockenem Stickstoff oder Edelgas (z.B. Argon) durch die Reaktionsmasse zu leiten. Der Fortgang der Reaktion kann durch die Bestimmung des NCO-Gehalts des Reaktionsgemischs kontrolliert werden. Die Reaktion wird im allgemeinen bei Erreichen eines Oligomerisierungsgrades von 5 bis 70, vorzugsweise 15 bis 40 %, abgebrochen. Unter "Oligomerisierungsgrad" ist hierbei der Prozentsatz der Isocyanatgruppen zu verstehen, die während der Umsetzung unter Dimerisierung oder Trimerisierung abreagieren. Der genannte Oligomerisierungsgrad entspricht einem NCO-Gehalt des Reaktionsgemisches von 47,5 bis 15, vorzugsweise 42,5 bis 30 Gew.-%.

Der Abbruch der Reaktion erfolgt durch Zugabe eines Katalysatorengifts, mit welchem die Wirkung des Katalysators aufgehoben wird. Geeignete Katalysatorengifte sind beispielsweise die in DE-OS 1 670 720 genannten Alkylierungs- oder Acylierungsmittel, Schwefel und insbesondere die in DE-OS 3 432 081 für diesen Zweck empfohlenen Sulfonylisocyanate. Gut geeignet sind auch Trialkylsiloxysulfonylisocyanate wie beispielsweise Trimethylsiloxysulfonylisocyanat. Das Katalysatorengift wird in einer, bezogen auf den Katalysator, zumindest halbmolaren Menge verwendet. Vorzugsweise liegt das Molverhältnis Katalysator:Katalysatorengift bei 1:0,5 bis 1:2.

Im Anschluß an den Abbruch der Reaktion wird das im Reaktionsgemisch vorliegende freie, nicht umgesetzte HDI durch eine geeignete Maßnahme, wie z.B. Extraktion (beispielsweise unter Verwendung von n-Hexan als Extraktionsmittel) oder vorzugsweise Dünnschichtdestillation, im Hochvakuum bei 110 bis 180°C, vorzugsweise wegen der empfindlichen Uretdiongruppen bei 110 bis 130°, bis auf einen Restgehalt von max. 0,5 Gew.-% entfernt.

Die erfindungsgemäßen Verfahrensprodukte zeichnen sich im Vergleich zu Produkten vom Stand der Technik durch eine geringere Verfärbung aus, weil beim erfindungsgemäßen Verfahren weniger Katalysator benötigt wird. Sie sind im allgemeinen farblose bis leicht gelb gefärbte Flüssigkeiten mit einer Farbzahl (HAZEN) nach DIN 53 409 von unter 200, meistens von unter 100. Sie besitzen eine Viskosität bei 23° C von 50 bis 3000 mPas. Sie haben einen NCO-Gehalt von etwa 10 bis 24 Gew.-%, bevorzugt von 18 bis 23 Gew.-%.

Während der erfindungsgemäßen Umsetzung findet gleichzeitig eine Dimerisierungsreaktion und eine Trimerisierungsreaktion statt. Bei den erfindungsgemäßen Verfahrensprodukten handelt es sich vor allem um Gemische von Uretdiongruppen aufweisenden Diisocyanaten und Isocyanuratgruppen aufweisenden Polyisocyanaten und, da die beiden Reaktionen statistisch ablaufen, geringen Anteilen an modifizierten Polyisocyanaten, die sowohl Uretdiongruppen als auch Isocyanuratgruppen aufweisen. Das Molverhältnis von Uretdion- zu Isocyanuratgruppen in den erfindungsgemäßen Verfahrensprodukten liegt bei 1:9 bis 9:1, im allgemeinen jedoch bei 1:3 bis 3:1. Es läßt sich während der Durchführung des erfindungsgemäßen Verfahrens innerhalb dieser Grenzen durch die Wahl des Katalysators und die Umsetzungstemperatur beeinflussen und beispielsweise durch Heißtitration mit Dibutylamin-Lösung und IR-spektroskopische Untersuchungen quantitativ bestimmen. Gegenüber den Verfahren des Standes der Technik hat das erfindungsgemäße Verfahren den Vorteil, daß es in kurzer Zeit, z.B. in weniger als einem Arbeitstag, unter sehr milden Bedingungen durchgeführt werden kann und deshalb auch sehr einfach kontinuierlich ausgestaltet werden kann. Es ist ein wesentlicher technischer Vorteil, daß in kleinen Apparaturen schnell eine große Produktmenge durchgesetzt werden kann.

Da bei der Durchführung des erfindungsgemäßen Verfahrens nur geringe Katalysatormengen eingesetzt werden, kann auch die Menge des Desaktivators, d.h. des Katalysatorengifts entsprechend gering gehalten werden, was zur Folge hat, daß die erfindungsgemäßen Verfahrensprodukte nur äußerst geringe Mengen an Nebenprodukten, die aus Katalysator und Katalysatorgift entstehen, enthalten, die gelöst bleiben und bei der späteren Verwendung der Verfahrensprodukte nicht stören. Auch bei Verwendung von technischem HDI, welches nicht vorab durch Destillation über schwach basischen Verbindungen wie Metalloxiden oder Natriumhydrogencarbonat einer üblichen Reinigungsoperation zwecks Entfernung von Spuren an chlorhaltigen Verbindungen unterzogen worden ist, werden klare und farblose Verfahrensprodukte erhalten. Wegen ihrer niedrigen Viskosität sind die Verfahrensprodukte insbesondere zur Herstellung von lösungsmittelfreien oder lösungsmittelarmen Zweikomponenten-Polyurethanlacken geeignet.

Bei der Verwendung der erfindungsgemäßen Verfahrensprodukte können diese in mit Blockierungsmitteln für Isocyanatgruppen blockierter Form verwendet werden. Geeignete Blockierungsmittel sind beispielsweise die in EP-A-10 589, Seite 15, Zeilen 14-26 beispielhaft genannten Verbindungen.

Die erfindungsgemäßen Verfahrensprodukte dienen, insbesondere in Kombination mit den aus der Polyurethanlacktechnik bekannten Polyhydroxypolyestern, Polyhydroxypolyethern und insbesondere Polyhydroxypolyacrylaten, zur Herstellung von hochwertigen Zweikomponenten-Polyurethanlacken, wobei diese Lacke neben den genannten höhermolekularen Polyhydroxylverbindungen auch niedermolekulare, vorzugsweise aliphatische Polyole enthalten können. Insbesondere Kombinationen der erfindungsgemäßen Verfahrensprodukte mit Polyhydroxypolyacrylaten, stellen wertvolle Zweikomponenten-Bindemittel für hochwertige, äußerst wetterbeständige Lacke dar.

Auch Polyamine, insbesondere in blockierter Form als Polyketimine oder Oxazolidine, sind denkbare

Reaktionspartner für die erfindungsgemäßen Verfahrensprodukte.

Die Mengenverhältnisse, in welchen die erfindungsgemäßen, gegebenenfalls blockierten Polyisocyanate und die genannten Reaktionspartner bei der Herstellung von Polyurethanlacken umgesetzt werden, werden im allgemeinen so gewählt, daß auf eine (gegebenenfalls blockierte) Isocyanatgruppe 0,8 bis 3, vorzugsweise 0,9 bis 1,8, Hydroxyl-, Amino- und/oder Carboxylgruppen entfallen.

Die Uretdiongruppe kann bekanntlich unter bestimmten Bedingungen ebenfalls als Reaktivgruppe eingesetzt werden und stellt gewissermaßen eine blockierte NCO-Gruppe dar. Bei Einbrennlacken, die bei erhöhter Temperatur ausgehärtet werden, kann die Uretdiongruppe praktisch als verkappte NCO-Gruppe in die Mengenverhältnisse von Polyisocyanat und Reaktionspartner mit einbezogen werden.

Zur Beschleunigung der Aushärtung können in bekannter Weise die in der Isocyanatchemie üblichen Katalysatoren verwendet werden, wie z.B. tert.-Amine wie Triethylamin, Pyridin, Methylpyridin, Benzyldimethylamin, N,N-Dimethylaminocyclohexan, N-Methylpiperidin, Pentamethyl-diethylentriamin, N,N'-Endoethylenpiperazin, N,N'-Dimethylpiperazin oder Metallsalze wie Eisen (III)-chlorid, Zinkchlorid, Zink-2-ethylcaproat, Zinn(II)-2-ethylcaproat, Dibutylzinn(IV)-dilaurat oder Molybdänglykolat.

In blockierter Form werden die erfindungsgemäßen Verfahrensprodukte in Kombination mit Polyhydroxylverbindungen der genannten Art, insbesondere zur Herstellung von Einbrennlacken, verwendet, die in Abhängigkeit vom eingesetzten Blockierungsmittel bei Temperaturen von 80 bis 180°C zu hochwertigen Lacküberzügen ausgehärtet werden können.

Zur Herstellung der gebrauchsfertigen Lacke werden gegebenenfalls blockiertes Polyisocyanat, Polyfunktioneller Reaktionspartner, gegebenenfalls Katalysator für die Isocyanat-Polyaddition und die üblichen Zusätze, wie z.B. Pigmente, Farbstoffe, Füllstoffe und Verlaufmittel miteinander auf einem üblichen Mischaggregat, wie z.B. auf einer Sandmühle, entweder mit oder ohne Lösungs- und Verdünnungsmittel, gut vermischt und homogenisiert.

Die Anstrich- und Überzugsmittel können in Lösung oder aus der Schmelze, oder in fester Form nach den üblichen Methoden wie z.B. Streichen, Rollen, Gießen oder Spritzen, auf den zu beschichtenden Gegenstand aufgebracht werden.

Die die erfindungsgemäßen Polyisocyanate enthaltenden Lacke ergeben Filme, die überraschend gut auf metallischem Untergrund haften, besonders lichtecht, wärmefarbstabil und sehr abriebfest sind. Darüber hinaus zeichnen sie sich durch große Härte, Elastizität, sehr gute Chemikalienbeständigkeit, hohen Glanz, ausgezeichnete Wetterbeständigkeit und gute Pigmentierbarkeit aus.

In den nachfolgenden Beispielen beziehen sich alle Prozentangaben und alle Angaben in "ppm" auf das Gewicht.

## Beispiele

### Beispiele 1 (erfindungsgemäß)

1200 g HDI werden bei ca. 20°C in einem Rührgefäß durch Anlegen von Vakuum (50 mbar) und kräftiges Rühren in ca. 10 Minuten entgast. Der Gasraum der Apparatur wird dann mit reinem Stickstoff gefüllt. Anschließend wird ein starker Strom von reinem, trockenem Stickstoff etwa 1 Stunde bei ca. 25°C durch die Flüssigkeit geleitet. Während das eingesetzte HDI einen $CO_2$-Gehalt von 56 ppm aufwies, war nach den genannten Maßnahmen der $CO_2$-Gehalt auf 5 ppm abgefallen. Während der gesamten weiteren Reaktion wird weiter Stickstoff durch die Reaktionsmasse geleitet.

Anschließend gibt man 1,5 g (ca. 0,007 Mol bzw. 0,125 %, bezogen auf HDI) Tributylphosphin in die auf 60°C erwärmte Flüssigkeit und rührt bei dieser Temperatur. Der Fortgang der Umsetzung wird durch Bestimmung des Isocyanatgehaltes kontrolliert. Nach 2 Stunden ist der NCO-Gehalt auf 38,6 % abgesunken (Oligomerisierungsgrad: ca. 22,8 %).

Man stoppt nun die Reaktion durch Zugabe von 1,4 g (ca. 0,007 Mol) Trimethylsiloxysulfonylisocyanat, rührt noch 30 Min. und destilliert dann in einem Kurzwegverdampfer bei 120°C und 0,01 mbar monomeres HDI ab.

Man erhält 474 g eines monomerenfreien Sumpfproduktes mit folgenden Eigenschaften:

| | | |
|---|---|---|
| Viskosität | : | 130 mPas/23° C |
| Farbzahl (HAZEN) nach DIN 53 409 | : | 90 |
| NCO-Gehalt | : | 21,4 % |
| Gehalt an freiem HDI: | : | 0,2 % |
| Molverhältnis von Uretdion- zu Isocyanuratgruppen ca. | : | 3:2 |

Beispiel 2 (Vergleichsversuch)

Man benutzt in einer entsprechenden Apparatur 1200 g des gleichen HDI wie in Beispiel 1, füllt den Reaktionsraum über der flüssigen Phase mit trockenem Stickstoff, verzichtet aber auf die Entfernung von im HDI gelösten $CO_2$, indem man nicht mit Stickstoff ausbläst.

Nun wird die Flüssigkeit auf 60°C erwärmt und mit 3 g (0,014 Mol bzw. 0,25 %, bezogen auf HDI) Tributylphosphin versetzt. Unter gutem Rühren wird die Umsetzung eingeleitet. Nach 2 Stunden ist der NCO-Gehalt erst auf 47,3 % abgefallen. Nach 8 Stunden auf 43,4 %, nach 13 Stunden auf 40,2 %. Nun wird die Reaktion durch Zugabe von 2,8 g Trimethylsilyloxylsulfonylisocyanat abgebrochen. Die weitere Verarbeitung erfolgt wie in Beispiel 1.

Man erhält 402 g einer viskosen Flüssigkeit mit folgenden Eigenschaften:

| | | |
|---|---|---|
| Viskosität | : | 120 mPas/23° C |
| Farbzahl (HAZEN) nach DIN 53 409 | : | 170 |
| NCO-Gehalt | : | 22,0 % |
| Gehalt an freiem HDI | : | 0,2 %. |

Ergebnis des Vergleichs:

Das erfindungsgemäße Verfahren arbeitet um ein vielfaches schneller mit weniger Katalysator, weniger Desaktivator und höherer Ausbeute. Das Endprodukt hat eine bessere Farbzahl.

Modifiziert man das Beispiel 1 so, daß man die Katalysatormenge so niedrig wählt wie in Beispiel 1, dauert die Umsetzung noch länger. Nach Erreichung eines NCO-Gehaltes von 42,0 % verändert sich der NCO-Gehalt nur noch minimal.

Beispiel 3

Das Beispiel 6 der DE-OS 3 432 081 wird nachgearbeitet und gemäß der vorliegenden Erfindung modifiziert. Zu 400 g HDI, das wie in Beispiel 1 von Kohlendioxid befreit wurde, gibt man 1 g frisch destilliertes Hexamethylphosphorigsäuretriamid (in Beispiel 6 der DE-OS 3 432 081 werden zu 400 g HDI 1 g Katalysator gegeben). Nach 45 Min. bei 60°C findet man einen NCO-Gehalt von 38,8 % (Vergleich: 40,0 %). Die Aufarbeitung ergibt 190 g eines hellgelb gefärbten Polyisocyanats (Vergleich: 152 g).

Der Vergleich zeigt auch hier, daß nach dem erfindungsgemäßen Verfahren mit weniger Katalysator eine bessere Ausbeute erzielt werden kann.

Beispiele 4 bis 8

Diese Beispiele geben die Variationsbreite des erfindungsgemäßen Verfahrens in bezug auf Katalysatormenge und Temperatur wieder. Durch Ausblasen mit Stickstoff bei 40 bis 60°C wird Kohlendioxid bis auf einen Restgehalt von 2 bis 10 ppm entfernt.

Die Ergebnisse sind in der Tabelle 1 zusammengefaßt. Es werden die Katalysatormenge, Temperatur und Dauer der Umsetzung sowie der NCO-Gehalt beim Abbruch der Reaktion angegeben. Tabelle 2 gibt die Eigenschaften des Endprodukts wieder.

Tabelle 1

| Beispiel | HDI (g) | $CO_2$-Gehalt nach Ausblasen mit $N_2$ | Katalysator Tributylphosphin | Umsetzungstemperatur (°C) | NCO-Gehalt bei Desaktivierung | Dauer der Umsetzung |
|---|---|---|---|---|---|---|
| 4 | 1600 | 2 ppm | 4 g (0,25 %) | 23 | 37,5 % | 9 h |
| 5 | 1600 | 7 ppm | 2 g (0,125 %) | 35 | 38,8 % | 5 h |
| 6 | 1600 | 10 ppm | 2 g (0,125 %) | 40 | 41,0 % | 1,5 h |
| 7 | 1600 | 2 ppm | 8 g (0,5 %) | 50 | 22,1 % | 6 h |
| 8 | 1600 | 4 ppm | 1 g (0,06 %) | 40 | 40,2 % | 10 h |

**Tabelle 2**

| Beispiel | Produktmenge nach Entfernung von HDI | NCO-Gehalt (%) | Viskosität/23° C (mPas) | Gehalt an freiem HDI (%) |
|---|---|---|---|---|
| 4 | 698 g | 21,1 | 160 | 0,4 |
| 5 | 625 g | 21,8 | 140 | 0,4 |
| 6 | 550 g | 22,6 | 130 | 0,2 |
| 7 | 1240 g | 15,5 | 15000 | 0,1 |
| 8 | 600 g | 21,8 | 140 | 0,05 |

## Patentansprüche

1. Verfahren zur Herstellung von Isocyanuratgruppen und Uretdiongruppen aufweisenden Polyisocyanatgemischen durch Oligomerisierung eines Teils der Isocyanatgruppen von Hexamethylendiisocyanat unter Verwendung von Trialkylphosphinen und/oder peralkylierten Phosphorigsäuretriamiden als die Dimerisierung und die Trimerisierung von Isocyanatgruppen beschleunigende Katalysatoren bis zum gewünschten Oligomerisierungsgrad, Beendigung der Reaktion durch Zugabe eines Katalysatorgifts und Entfernung von nicht umgesetztem Hexamethylendiisocyanat bis auf einen Restgehalt von max. 0,5 Gew.-%, dadurch gekennzeichnet, daß man das zum Einsatz gelangende Hexamethylendiisocyanat vor der Zugabe des Katalysators bis auf einen Restgehalt von weniger als 10 ppm (Gewicht) von Kohlendioxid befreit.

2. Verfahren gemäß Anspruch 1, dadurch gekennzeichnet, daß man das zum Einsatz gelangende Hexamethylendiisocyanat bei 0 bis 120°C durch Durchleiten eines Stickstoff- oder Edelgasstroms von Kohlendioxid befreit, bis der Restgehalt an Kohlendioxid unterhalb ppm (Gewicht) abgesunken ist.

3. Verfahren gemäß Anspruch 1 und 2, dadurch gekennzeichnet, daß man als Katalysator Tri(n-butyl)-phosphin oder Tris-(dimethylamino)-phosphin verwendet.

## Revendications

1. Procédé de production de mélanges de polyisocyanates présentant des groupes isocyanurate et des groupes uret-dione par oligomérisation d'une partie des groupes isocyanate de l'hexaméthylènediisocyanate avec utilisation de trialkylphosphines et/ou de triamides d'acide phosphoreux peralkylés comme catalyseurs accélérant la dimérisation et la trimérisation de groupes isocyanate jusqu'au degré désiré d'oligomérisation, arrêt de la réaction par addition d'un poison du catalyseur et élimination de l'hexaméthylènediisocyanate n'ayant pas réagi jusqu'à une teneur résiduelle de 0,5 % en poids au maximum, caractérisé en ce que l'hexaméthylènediisocyanate utilisé est débarrassé de l'anhydride carbonique jusqu'à une teneur résiduelle de moins de 10 ppm (en poids) avant l'addition du catalyseur.

2. Procédé suivant la revendication 1, caractérisé en ce qu'on débarrasse de l'anhydride carbonique l'hexaméthylènediisocyanate utilisé par passage, à 0-120°C, d'un courant d'azote ou de gaz noble, jusqu'à ce que la teneur résiduelle en anhydride carbonique se soit abaissée au-dessous de 10 ppm (en poids).

3. Procédé suivant les revendicaticns 1 et 2, caractérisé en ce qu'on utilise comme catalyseur la tri(n-butyl)phosphine ou la tris-(diméthylamino)-phosphine.

## Claims

1. A process for the production of polyisocyanate mixtures containing isocyanurate groups and uretdione groups by partial oligomerization of the isocyanate groups of hexamethylene diisocyanate using trialkyl phosphines and/or peralkylated phosphorous acid triamides as the catalysts accelerating the dimerization and trimerization of isocyanate groups to the desired degree of oligomerization, termination of the reaction by addition of a catalyst poison and removal of unreacted hexamethylene diisocyanate to a residual content of at most 0.5% by weight, characterized in that, before addition of the catalyst, the hexamethylene diisocyanate used is freed from carbon dioxide to a residual content of less than 10 ppm (weight).

2. A process as claimed in claim 1, characterized in that the hexamethylene diisocyanate used is freed from carbon dioxide by passing a stream of nitrogen or noble gas through at 0 to 120°C until the residual carbon dioxide content is less than 10 ppm (weight).

3. A process as claimed in claims 1 and 2, characterized in that tri-(n-butyl)-phosphine or tris-(dimethylamino)phosphine is used as the catalyst.